# EUROPÄISCHE PATENTSCHRIFT

(11) **EP 2 218 704 B1**
(45) Veröffentlichungstag und Bekanntmachung des Hinweises auf die Patenterteilung: **25.02.2015**
(21) Anmeldenummer: 09002122.1
(22) Anmeldetag: 16.02.2009
(51) Int. Cl.: C05F 17/02

(54) **Fermentationsanlage und Verfahren zum Betreiben einer solchen**
Fermentation assembly and method for operating the same
Installation de fermentation et son procédé de fonctionnement

(43) Veröffentlichungstag der Anmeldung: 18.08.2010
(73) Patentinhaber: KOMPOFERM GmbH, 33428 Marienfeld (DE)
(72) Erfinder: Eggersmann, Karlgünter, 33428 Marienfeld (DE)
(74) Vertreter: Schober, Mirko

(56) Entgegenhaltungen:
- EP-A- 0 501 319
- EP-A- 0 799 812
- EP-A- 1 681 344
- EP-A- 1 970 435
- WO-A-02/06439
- WO-A1-2009/000309
- DE-A1-102004 009 161
- DE-A1-102007 015 981
- ES-A1- 2 245 210
- GB-A- 717 785
- JP-A- 8 034 523

## Beschreibung

Die Erfindung betrifft eine Fermentationsanlage nach dem Oberbegriff des Anspruchs 1 und ein Verfahren zum Betreiben einer solchen.

Bei entsprechenden Anlagen werden üblicherweise sogenannte Fermenter in Form von Garagen oder Boxen eingesetzt, die mit Fermentationsmaterial beschickt werden. Hierzu wird üblicherweise an der Stirnseite eine Öffnung vorgesehen, durch welche die Fermenterboxen mittels eines Radladers beschickt werden.

Diese Art der Beschickung ist uneffektiv und aufwendig und erlaubt insbesondere keinen hohen Automatisierungsgrad. Zudem sammelt sich im Fermenter im Raum über dem Fermentationsmaterial durch vorhandene Luft so viel Stickstoff, dass es erforderlich ist, diesen Raum zumindest zu Beginn des Fermentationsprozesses zu spülen, damit die Stickstoffkonzentration einen kritischen Wert nicht übersteigt, was für aus dem Prozess gewonnenes Biogas nachteilig wäre. Das Spülen und Abführen von Stickstoff erfordert anlagentechnisch erhöhten Aufwand, weil außerhalb der Fermenter in der Anlage dafür vorgesehene Apparaturen und Anlagenteile vorgesehen werden müssen, die letztlich einen Teil der Kosten der Gesamtanlage ausmachen.

Der Erfindung liegt die Aufgabe zugrunde, eine Fermentationsanlage und ein entsprechendes Verfahren der eingangs genannten Art anzugeben, mit denen die erwähnten Nachteile nicht auftreten.

Gelöst wird diese Aufgabe durch eine Fermentationsanlage mit den Merkmalen des Anspruchs 1 sowie ein Verfahren zum Betreiben einer solchen mit den Merkmalen des Anspruchs 3. Vorteilhafte Ausführungsformen finden sich in den Unteransprüchen.

Erfindungsgemäß ist vorgesehen, die Beschickung und Verteilung im Fermenter mittels einer Verteileinrichtung gemäß der Definition im Anspruch 1 vorzunehmen, welche in Längsrichtung des Fermenters verschieblich ist. Hierdurch kann das Volumen oberhalb des Fermentationsmaterials im Fermenter verringert und damit das Verhältnis des Volumens des Fermentationsmaterials zum Gesamtvolumen erhöht werden. Gleichzeitig sinkt dadurch das Spülvolumen, also das Volumen, welches über kostenaufwendige Spüleinrichtungen aufgrund des oben erwähnten Stickstoffeintrags ausgetauscht werden muss. Mit der Erfindung ist es möglich, das Volumen oberhalb des Fermentationsmaterials so zu reduzieren, dass die Konzentration des Stickstoffs bezogen auf das Gesamtvolumen des gewonnenen Biogases unter 1% sinkt, so dass auf eine Spülung verzichtet werden kann.

Die Erfindung wird nachfolgend anhand der Figuren 1 bis 7 schematisch näher erläutert.
- Figur 1 -: zeigt eine Draufsicht auf einen Teil einer erfindungsegemäßen Fermentationsanlage,
- Figur 2 -: ist eine Ausschnittvergrößerung eines Teils von Figur 1,
- Figur 3 -: zeigt eine Schnittansicht durch einen unbefüllten Fermenter der in Figur 1 gezeigten Anlage,
- Figur 4 -: zeigt eine Schnittansicht durch einen erfindungsgemäß befüllten Fermenter,
- Figur 5 -: zeigt eine Querschnittansicht durch die Fermenter,
- Figur 6 -: zeigt eine zeigt Draufsicht auf einen Teil einer erfindungsgemäßen Fermentationsanlage mit einer alternativ ausgeführten Verteileinrichtung,
- Figur 7 -: zeigt eine Längsschnittansicht durch einen mit der alternativen Verteileinrichtung befüllten Fermenter.

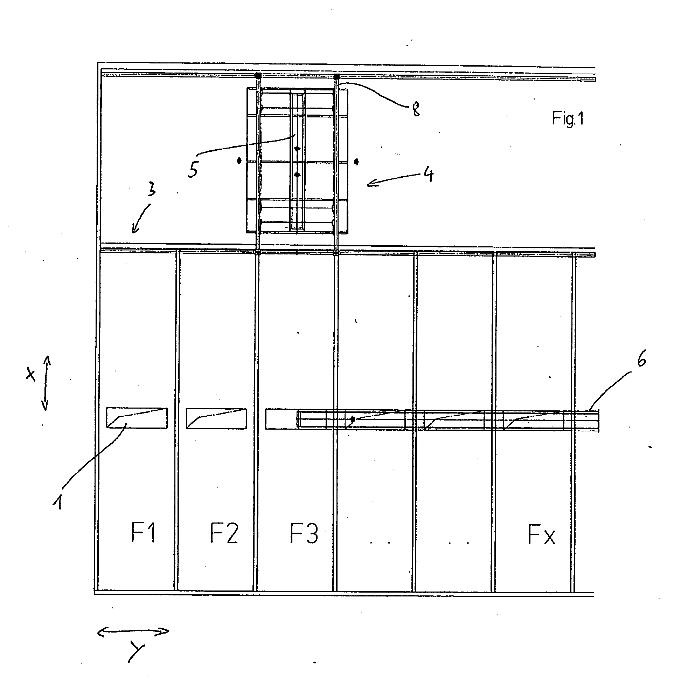

In Figur 1 ist ein typischer Aufbau einer erfindungsgemäßen Fermentationsanlage gezeigt. Diese weist in der Regel ein oder - wie gezeigt - mehrere Fermenter F1-Fx auf, welche bevorzugt als garagenförmige Fermenterboxen ausgebildet sind. Die Fermenter F1-Fx weisen in ihrem Dachbereich Beschickungsöffnungen 1 auf, durch welche das einzulagernde Fermentationsmaterial aus einer bevorzugt als Bandförderer ausgebildeten Beschickungsvorrichtung 6 in das Innere des Fermenters eingebracht werden kann. Parallel zu den Stirnseiten 3 der Fermenter F1-Fx ist eine Verteileinrichtung 4 in Richtung Y mittels eines Transportgestells 8 verfahrbar und kann so vor der stirnseitigen Öffnung (Pos. 3) eines ausgewählten zu beschickenden Fermenters F1-Fx positioniert werden. Die Verteileinrichtung ist so ausgebildet, dass sie aus dem Transportgestell 8 in Längsrichtung X der Fermenter F1-Fx in diese einbringbar ist. Die Verteileinrichtung 4 weist ein Förderelement 5 auf, welches das im Fermenter F1-Fx einzulagernde Fermentationsmaterial von der Beschickungsvorrichtung 6 übernimmt und im Fermenter F1-Fx abwirft.

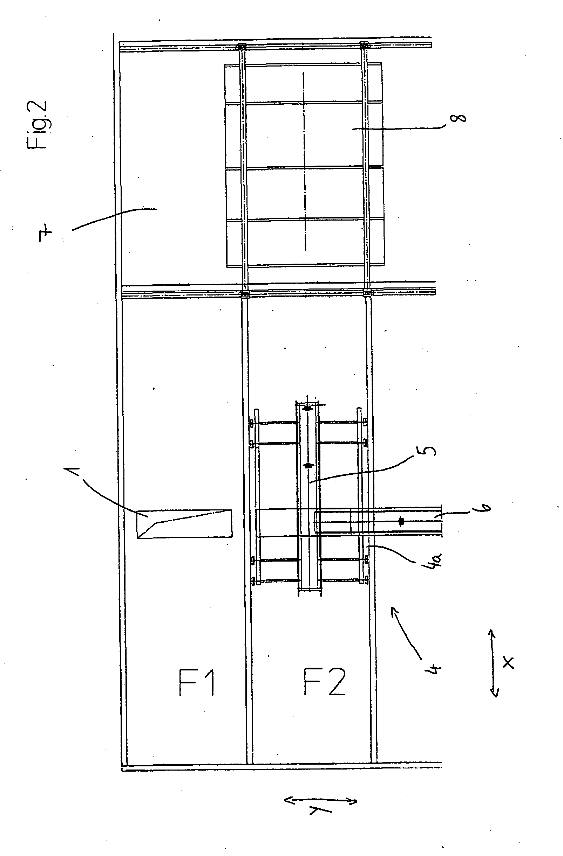

Diese Situation ist in der Ausschnittvergrößerung in Figur 2 gezeigt. Die Transportvorrichtung 8 ist in einem Korridor 7 verschieblich und wird vor den Fermenter F2 gefahren, die Verteileinrichtung 4 wird in X-Richtung in den Fermenter F2 eingefahren, so dass ein Abschnitt der Fördereinrichtung 5 unterhalb der Beschickungsöffnung 1 liegt. Die Beschickungsvorrichtung 6 wird mit ihrem Abwurfende über die Öffnung 1 platziert, so dass Fermentationsmaterial von der Beschickungsvorrichtung 6 durch die Öffnung 1 auf die Fördereinrichtung 5 fällt. Die Fördereinrichtung ist zusammen mit der Verteileinrichtung in Längsrichtung X des Fermenters F2 beweglich, zudem lässt sich die Fördereinrichtung 5 quer zur Längsrichtung an der Verteileinrichtung 4 verschieben, so dass beim Befüllen des Fermenters jeder Punkt im Fermenter F2 angefahren werden kann, so dass eine homogene Verteilung des Fermentationsmaterials im Fermenter F2 möglich ist. Zudem ist der Beschickungsprozess mit dieser Maßnahme vollständig automatisierbar. Bevorzugt kann die Fördereinrichtung 5 in zwei Richtungen bewegt werden.

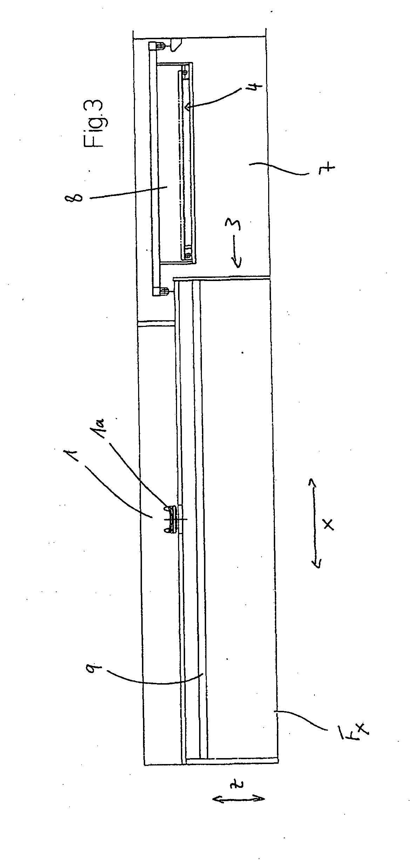

Figur 3 zeigt den leeren Fermenter unmittelbar vor der Beschickung. Die Transporteinrichtung 8 befindet sich mit der Verteileinrichtung 4 im Korridor 7 vor dem Fermenter Fx. Im Fermenter Fx ist bevorzugt in Höhe der Verteileinrichtung 4 eine Laufschiene 9 vorgesehen, auf der die Verteileinrichtung 4 in Längsrichtung X des Fermenters Fx in diesem hin und her bewegt werden kann.

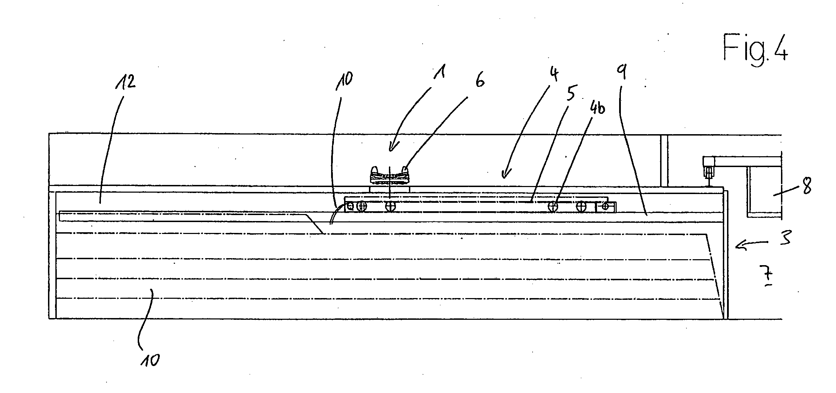

Figur 4 zeigt den Befüllvorgang im Längsschnitt durch den Fermenter. Die Verteileinrichtung 4 weist Rollen 4b auf, mit denen sie auf den Laufschienen 9 aufliegt. Das aus der Beschickungseinrichtung 6 durch die Beschickungsöffnung 1 abgeworfene Fermentationsmaterial 10 gelangt auf die Fördereinrichtung 5 und von deren Endabschnitt in den Fermenter. Dabei wird die Fördereinrichtung 5 im Fermenter in einer Ebene parallel zum Boden verschoben, so dass eine gleichmäßige Verteilung des Fermentionsmaterials 10 im Fermenter möglich ist.

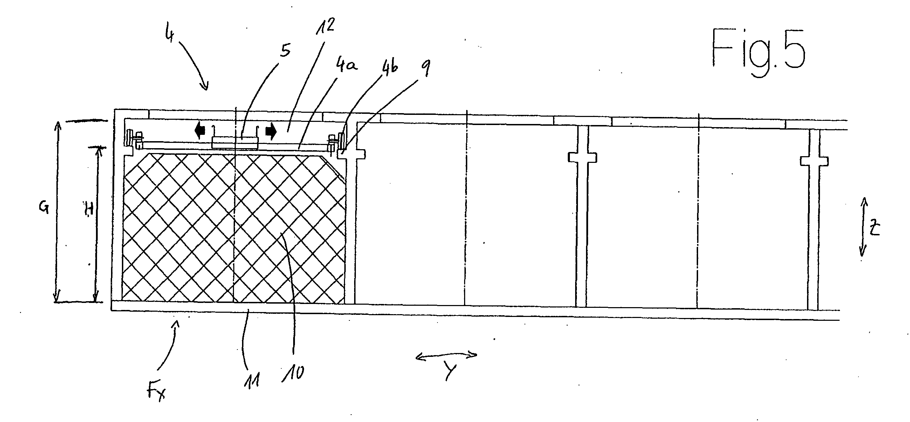

Wie in Figur 5 gezeigt, kann durch die flache Bauweise der Verteileinrichtung 4 der nicht mit dem Fermentationsmaterial 10 befüllbare Raum 12 im Gegensatz zu einer Radladerbeschickung verringert werden. So ist mit diesem Verfahren eine Verringerung des Spülvolumens 12 möglich, da das Fermentationsmaterial 10 bis zu einer Höhe in den Fermenter eingebracht werden kann, die über dem 0,7-fachen der Gesamtinnenhöhe G des Fermenters liegt. Der im Prozess vorhandene Stickstoff kann aufgrund des geringen Spülvolumens 12 daher im Vergleich zu herkömmlichen Verfahren deutlich reduziert werden.

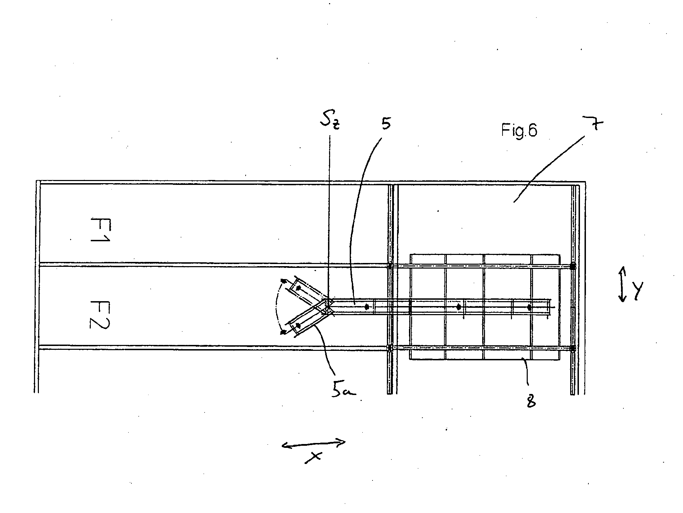

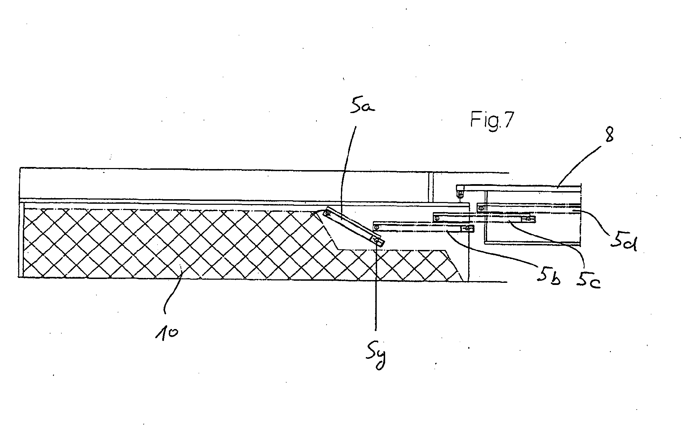

Die Verteilung des Fermentationsmaterials im Fermenter erfolgt durch eine modifizierte Fördereinrichtung 5 gemäß der Definition im Anspruch 1, die in den Figuren 6 und 7 schematisch dargestellt wird. Die Fördereinrichtung 5 weist in Längsrichtung X des Fermenters teleskopierbare Förderabschnitte 5b, 5c, 5d auf, welche gegenüber der Transporteinrichtung 8 verschieblich sind und in den Fermenter hinein ausgefahren werden können. Am zum Fermenter hin gerichteten Ende der Fördereinrichtung 5 ist ein verschwenkbarer Abwurfabschnitt 5a vorgesehen, der sich um eine Achse Sy parallel zum Boden 11 bzw. senkrecht zur Achse Sz des Fermenters und um eine Achse Sz vertikal zum Fermenterboden verschwenken lässt, so dass zusammen mit der Verstellung der Fördereinrichtung in X-Richtung jeder Punkt im Fermenter mit dem Abwurfabschnitt 5a erreicht werden kann. Durch diese Maßnahme ist es möglich, die Beschickung ausschließlich von der Stirnseite 3 des Fermenters durchzuführen, Beschickungsöffnungen im Dach des Fermenters sind dann nicht erforderlich.

## Patentansprüche

1. Fermentationsanlage mit wenigstens einem Fermenter (F1, F2, ..., Fx) zur Aufnahme von Fermentationsmaterial, wobei der wenigstens eine Fermenter (F1, F2, ..., Fx) ein boxenförmiger Fermenter (F1, F2, ..., Fx) mit wenigstens einer Öffnung (2) an der Stirnseite (3) ist, durch welche eine Verteileinrichtung (4) zum Verteilen von einzubringendem Fermentationsmaterial (10) in den Fermenter (F1, F2, ..., Fx) eingebracht und aus diesem entfernt werden kann, wobei die Verteileinrichtung (4) in Längsrichtung des Fermenters (F1, F2, ..., Fx) verschieblich gelagert ist und ein Förderelement (5) aufweist, welches das Fermentationsmaterial aufnimmt und an einer vorgegebenen Stelle im Fermenter (F1, F2, ..., Fx) abwirft, wobei das Förderelement (5) oder die Verteileinrichtung (4) in wenigstens einer Ebene parallel zum Boden des Fermenters (F1, F2, ..., Fx) beweglich gelagert ist,
**dadurch gekennzeichnet,**
**dass** die Fermentationsanlage eine Transportvorrichtung (8) aufweist, mittels derer die Verteileinrichtung (4) in einer Richtung (Y) senkrecht zur Längsrichtung (X) des Fermenters (F1, F2, ..., Fx) verfahrbar ist, und das Förderlement (5) in Längsrichtung (X) des Fermenters (F1, F2, ..., Fx) teleskopierbare Förderabschnitte (5b, 5c, 5d) aufweist, welche gegenüber der Transportvorrichtung (8) verschieblich sind und in den Fermenter (F1, F2, ..., Fx) hinein ausfahrbar sind, und wobei das Förderelement (5) einen schwenkbaren Abwurfabschnitt (5a) aufweist, der um eine erste Achse (Sz) senkrecht zum Boden (11) des Fermenters (F1, F2, ..., Fx) schwenkbar gelagert ist.

2. Fermentationsanlage nach Anspruch 1,
**dadurch gekennzeichnet,**
**dass** der Abwurfabschnitt (5a) auch um eine Schwenkachse (Sy) senkrecht zur ersten Achse (Sz) verschwenkbar ist.

3. Verfahren zum Betreiben einer Fermentationsanlage, insbesondere nach einem der vorherigen Ansprüche, bei welchem wenigstens ein Fermenter (F1, F2, ..., Fx) zur Aufnahme von Fermentationsmaterial eingesetzt wird, wobei vor dem Beginn des Fermentationsprozesses der Fermenter (F1, F2, ..., Fx) mit Fermentationsmaterial bis zu einer vorgegebenen Höhe (H) unterhalb der Gesamtinnenhöhe (G) des Fermenters (F1, F2, ..., Fx) beschickt wird, wobei die vorgegebene Höhe (H) so gewählt ist, dass die im anschließenden Fermentationsprozess vorhandene Menge an Stickstoff bzw. stickstoffhaltigen Substanzen einen vorgegebenen Grenzwert nicht übersteigt,
**dadurch gekennzeichnet,**
**dass** die Verteilung des Fermentationsmaterials im Fermenter mittels einer Fördereinrichtung (5) erfolgt, welche in Längsrichtung (X) des Fermenters (F1, F2, ..., Fx) teleskopierbare Förderabschnitte (5b, 5c, 5d) aufweist, welche gegenüber einer die Verteileinrichtung (4) in einer Richtung (Y) senkrecht zur Längsrichtung (X) des Fermenters (F1, F2, ..., Fx) verfahrenden Transportvorrichtung (8) verschieblich und in den Fermenter (F1, F2, ..., Fx) hinein ausfahrbar sind, und wobei das Förderelement (5) einen schwenkbaren Abwurfabschnitt (5a) aufweist, der um eine erste Achse (Sz) senkrecht zum Boden (11) des Fermenters (F1, F2, ..., Fx) schwenkbar gelagert ist.

4. Verfahren nach Anspruch 3,
**dadurch gekennzeichnet,**
**dass** das Verhältnis der vorgegebenen Höhe (H) zur Gesamtinnenhöhe (G) größer als 0,7 beträgt.

## Claims

1. Fermentation assembly with at least one fermenter (F1, F2, ..., Fx) for receiving fermentation material wherein the at least one fermenter (F1, F2,..., Fx) is a box-like fermenter (F1, F2, ...,Fx) with at least one opening (2) on the end side (3) through which a distributor device (4) for distributing the fermentation material (10), which is to be introduced, can be inserted into the fermenter (F1, F2,..., Fx) and can be removed therefrom, wherein the distributor device (4) is mounted displaceable in the longitudinal direction of the fermenter (F1, F2, ..., Fx) and has a conveying element (5) which receives the fermentation material and discharges it at a predetermined place in the fermenter (F1, F2, ..., Fx), wherein the conveying element (5) or the distributor device (4) is mounted movable in at least one plane parallel to the base of the fermenter (F1, F2, ..., Fx),
**characterised in that**
the fermentation assembly has a transport device (8) by means of which the distributor device (4) is movable in a direction (Y) perpendicular to the longitudinal direction (X) of the fermenter (F1, F2, ..., Fx), and the conveying element (5) has conveying portions (5b, 5c, 5d) which are telescopic in the longitudinal direction (X) of the fermenter (F1, F2, ..., Fx) and which are displaceable relative to the transport device (8) and can be extended into the fermenter (F1, F2, ..., Fx), and wherein the conveying element (5) has a pivotal discharge portion (5a) which is mounted pivotal about a first axis (Sz) perpendicular to the base (11) of the fermenter (F1, F2,... , Fx).

2. Fermentation assembly according to claim 1
**characterised in that**
the discharge portion (5a) is also able to pivot about a pivotal axis (Sy) perpendicular to the first axis (Sz).

3. Method for operating a fermentation assembly, more particularly according to one of the preceding claims, wherein at least one fermenter (F1, F2,..., Fx) is used for receiving fermentation material, wherein before the start of the fermentation process the fermenter (F1, F2,..., Fx) is loaded with fermentation material up to a predetermined level (H) below the overall internal height (G) of the fermenter (F1, F2, ..., Fx), wherein the predetermined level (H) is selected so that the quantity of nitrogen or nitrogen-containing substances present in the subsequent fermentation process does not exceed a predetermined boundary value,
**characterised in that**
the distribution of the fermentation material in the fermenter takes place by means of a conveying device (5) which has conveying portions (5b, 5c, 5d) which are telescopic in the longitudinal direction (X) of the fermenter (F1, F2,..., Fx) and which are displaceable opposite a transport device (8) which moves the distributor device (4) in a direction Y perpendicular to the longitudinal direction (X) of the fermenter (F1, F2, ..., Fx), and which can be extended into the fermenter (F1, F2, ..., Fx), and wherein the conveyor element (5) has a pivotal discharge portion (5a) which is mounted pivotable about a first axis (Sz) perpendicular to the base (11) of the fermenter (F1, F2, ..., Fx).

4. Method according to claim 3
**characterised in that**
the ratio of the predetermined level (H) to the overall internal height (G) is greater than 0.7

## Revendications

1. Installation de fermentation comprenant au moins un fermenteur (F1, F2, ..., Fx) destiné à recevoir du matériau de fermentation, sachant que le fermenteur (F1, F2, ..., Fx), au moins prévu, est un fermenteur (F1, F2, ..., Fx) en forme de box doté, sur le côté frontal (3), d'au moins une ouverture (2), par laquelle un dispositif de distribution (4), servant à répartir le matériau de fermentation amené, peut être introduit dans le fermenteur (F1, F2, ..., Fx), et en être retiré, sachant que le dispositif de distribution (4) est monté de manière à pouvoir être déplacé dans la direction longitudinale du fermenteur (F1, F2, ..., Fx) et présente un élément de transport (5), qui prend en charge le matériau de fermentation et le décharge dans le fermenteur (F1, F2, ..., Fx), à un endroit prédéterminé, sachant que l'élément de transport (5) ou le dispositif de distribution (4) est monté, mobile, parallèlement par rapport au fond du fermenteur (F1, F2, ..., Fx), au moins sur un plan,
**caractérisée en ce que**
l'installation de fermentation présente un dispositif de transport (8) au moyen duquel le dispositif de distribution (4) peut être déplacé dans une direction (Y) perpendiculaire à la direction longitudinale (X) du fermenteur (F1, F2, ..., Fx), et que l'élément de transport (5) est doté de sections de transport (5b, 5c, 5d), qui, pouvant être télescopées dans la direction longitudinale (X) du fermenteur (F1, F2, ..., Fx), peuvent être déplacées par rapport au dispositif de transport (8) et introduites dans le fermenteur (F1, F2, ..., Fx), et sachant que l'élément de transport (5) présente une section de déchargement pivotante (5a) qui est montée, à pivotement autour d'un premier axe (Sz), perpendiculairement par rapport au fond (11) du fermenteur (F1, F2, ..., Fx).

2. Installation de fermentation selon la revendication 1,
**caractérisée en ce que**
la section de déchargement (5a) pivote également autour d'un axe de pivotement (Sy), perpendiculaire au premier axe (Sz).

3. Procédé de fonctionnement d'une installation de fermentation, en particulier selon l'une des revendications précédentes, dans lequel au moins un fermenteur (F1, F2, ..., Fx) est utilisé pour recevoir le matériau de fermentation, sachant que, avant le début du processus de fermentation, ledit fermenteur (F1, F2, ..., Fx) est rempli de matériau de fermentation jusqu'à une hauteur prédéterminée (H), au-dessous de la hauteur totale (G) dudit fermenteur (F1, F2, ..., Fx), sachant que la hauteur prédéterminée (H) est choisie de sorte que la quantité d'azote, respectivement de substances azotées, présentes pendant le processus de fermentation faisant suite au remplissage, ne soit pas supérieure à une valeur limite prédéterminée,
**caractérisé en ce que**,
la distribution du matériau de fermentation dans le fermenteur (F1, F2, ..., Fx) est effectuée au moyen d'un dispositif de transport (5) qui est doté de sections de transport (5b, 5c, 5d), qui peuvent être télescopées dans la direction longitudinale (X) du fermenteur (F1, F2, ..., Fx), peuvent être déplacées par rapport à un dispositif de transport (8), qui conduit le dispositif de distribution (4) dans une direction (Y) perpendiculaire à la direction longitudinale (X) du fermenteur (F1, F2, ..., Fx), et introduites dans le fermenteur (F1, F2, ..., Fx), et sachant que l'élément de transport (5) présente une section de déchargement pivotante (5a) qui est montée, à pivotement autour d'un premier axe (Sz), perpendiculairement par rapport au fond (11) du fermenteur (F1, F2, ..., Fx).

4. Procédé selon la revendication 3,
**caractérisé en ce que**
le rapport entre la hauteur (H) prédéterminée et la hauteur intérieure, totale (G) est supérieur à 0,7.
